# EUROPEAN PATENT APPLICATION

(11) **EP 4 431 100 A1**
(43) Date of publication of application: **18.09.2024**
(21) Application number: 22892741.4
(22) Date of filing: 08.11.2022
(51) Int. Cl.: A61K 31/7068, A61P 31/12, A61P 31/14, C07H 19/067

(54) **ANTIVIRAL AGENT**

(30) Priority: 12.11.2021 JP 2021184546; 25.03.2022 JP 2022050466
(71) Applicant: National University Corporation Hokkaido University, Sapporo-shi, Hokkaido 060-0808 (JP)
(72) Inventor: MAENAKA Katsumi, Sapporo-shi, Hokkaido 060-0808 (JP); MATSUDA Akira, Sapporo-shi, Hokkaido 060-0808 (JP); SAWA Hirofumi, Sapporo-shi, Hokkaido 060-0808 (JP); ORBA Yasuko, Sapporo-shi, Hokkaido 060-0808 (JP); SASAKI Michihito, Sapporo-shi, Hokkaido 060-0808 (JP); UEMURA Kentaro, Sapporo-shi, Hokkaido 060-0808 (JP)
(74) Representative: Cabinet Becker et Associés
(86) International application number: PCT/JP2022/041473
(87) International publication number: WO 2023/085242

(57) **Abstract**

The present invention provides an antiviral agent formed from a compound represented by general formula (A-1) or (A-2) shown below [wherein in the formulas, R¹ represents O, S, Se or =N-OR¹¹; R² represents O, S, Se or =N-OR¹¹; R³ represents -OR¹², -S-R¹³ or a guanidino group; R¹¹ represents H, an alkyl group or an aryl group; R¹² and R¹³ each independently represent an alkyl group or an aryl group; R⁴ represents H, - CO-R¹⁴ or -O-P(OH)(=O)-O-R¹⁵; R¹⁴ and R¹⁵ each independently represent an alkyl group or an aryl group; R⁵ and R⁶ each independently represent H or -CO-R¹⁶; and R¹⁶ represents an alkyl group of 1 to 6 carbon atoms or an aryl group], a salt of the compound, or a solvate of the compound or salt.

## Description

### [Technical Field]

The present invention relates to an antiviral agent that is effective as a therapeutic agent against infectious diseases caused by enveloped viruses having a positive-sense single-stranded RNA gene, such as coronaviruses and flaviviruses.

### [Background Art]

Coronaviruses are enveloped viruses having a positive-sense single-strand RNA gene. Examples of coronaviruses that infect humans include the virus that causes the common cold, as well as other viruses that cause acute respiratory syndrome. Examples of these other viruses include SARS-CoV-1 that caused the severe acute respiratory syndrome (SARS) that was widespread in 2003, MERS-CoV that caused the Middle East respiratory syndrome outbreak in 2013, and SARS-CoV-2 which causes the novel coronavirus COVID-19 pandemic that has raged since 2019. In particular, there are still few therapeutic drugs for COVID-19, and the development of more effective drugs is required.

One example of a therapeutic drug for COVID-19 is remdesivir. Remdesivir (GS-5734) is a monophosphoramidate prodrug of GS-441524, is an antiviral agent formed from a nucleic acid compound that was developed as a therapeutic drug for Ebola hemorrhagic fever and Marburg virus infections, and exhibits antiviral activity against single-stranded RNA viruses. GS-441524 is also expected to offer a therapeutic effect against COVID-19 (Non-Patent Document 1).

### [Citation List]

### [Non-Patent Document]

[Non-Patent Document 1]
Yan and Muller, ACS Medicinal Chemistry Letters, 2020, vol. 11, pp.1361 to 1366.

### [Summary of Invention]

### [Technical Problem]

The present invention has an object of providing an antiviral agent that is effective as a therapeutic drug for viral infectious diseases including COVID-19.

### [Solution to Problem]

Using a compound library based on nucleic acid compounds held by the Center for Research and Education on Drug Discovery, Faculty of Pharmaceutical Sciences, Hokkaido University, National University Corporation, the inventors of the present invention conducted screening for compounds having antiviral activity against dengue virus, which represents one enveloped virus having a positive-sense single-stranded RNA gene, and by selecting those compounds having high antiviral activity, were able to complete the present invention.

In other words, the present invention provides the antiviral agent and the like described below.
[1] An antiviral agent formed from a compound represented by general formula (A-1) or (A-2) shown below, a salt of the compound, or a solvate of the compound or salt. 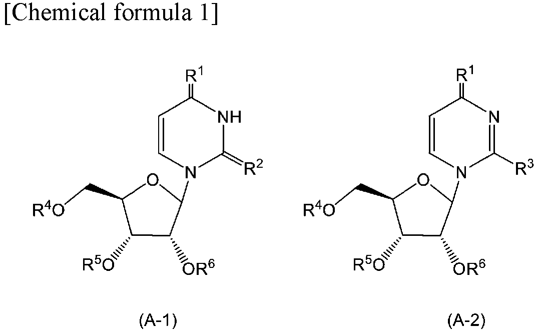 [In the formulas, R¹ represents an oxygen atom, sulfur atom, selenium atom, or =N-OR¹¹; R² represents an oxygen atom, sulfur atom, selenium atom, or =N-OR¹¹; R³ represents - O-R¹², -S-R¹³, or a guanidino group; R¹¹ represents a hydrogen atom, an alkyl group of 1 to 6 carbon atoms, or an aryl group; R¹² and R¹³ each independently represent an alkyl group of 1 to 6 carbon atoms or an aryl group; R⁴ represents a hydrogen atom, -CO-R¹⁴, or -O-P(OH)(=O)-O-R¹⁵; R¹⁴ and R¹⁵ each independently represent an alkyl group of 1 to 30 carbon atoms which may be substituted with a phenyl group (and when the alkyl group has 2 or more carbon atoms, an oxygen atom of an ether bond may exist between the carbon atoms) or an aryl group; R⁵ and R⁶ each independently represent a hydrogen atom or -CO-R¹⁶; and R¹⁶ represents an alkyl group of 1 to 6 carbon atoms or an aryl group.]
[2] The antiviral agent according to [1], wherein the compound represented by general formula (A-1) or (A-2) shown above is a compound represented by any of general formulas (1) to (9) shown below. 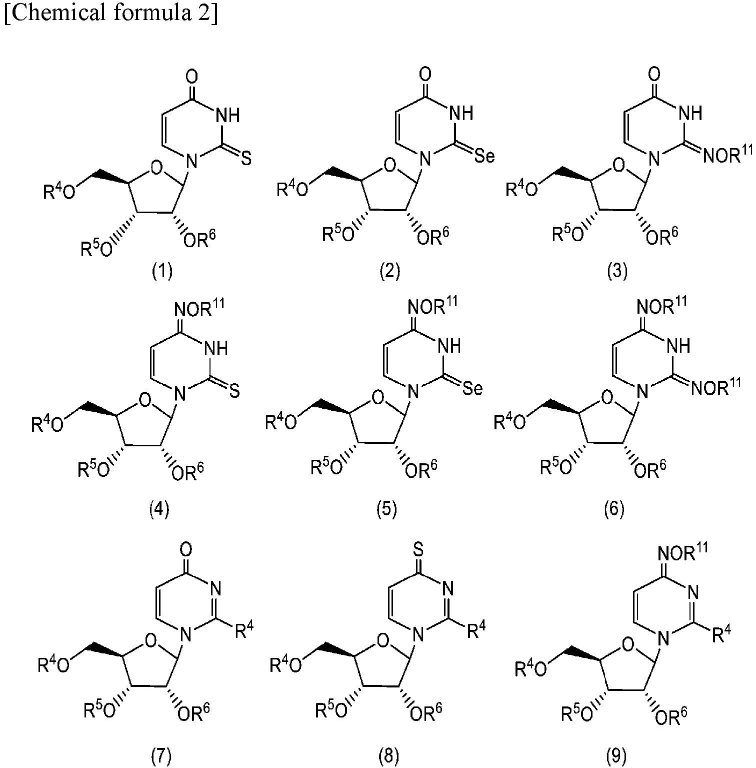
[3] The antiviral agent according to [1], wherein the compound represented by general formula (A-1) or (A-2) shown above is a compound represented by any of formulas (A1), (A5), (A6), (A7) and (A8) shown below. 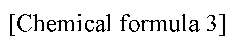 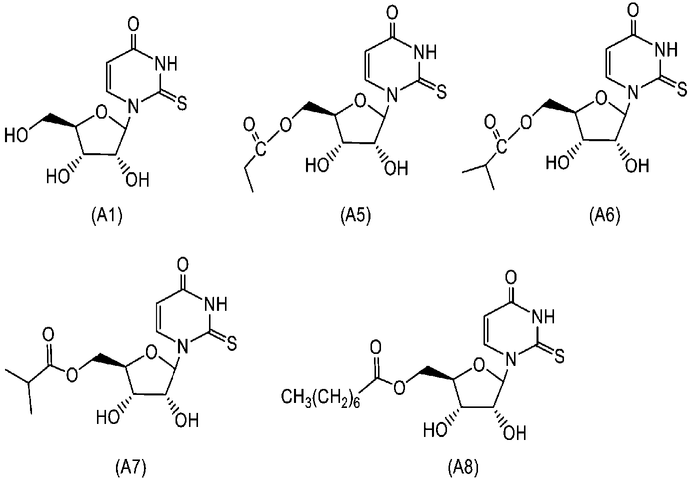
[4] The antiviral agent according to [1], wherein the compound represented by general formula (A-1) or (A-2) shown above is a compound represented by any of formulas (A2) to (A4) shown below. 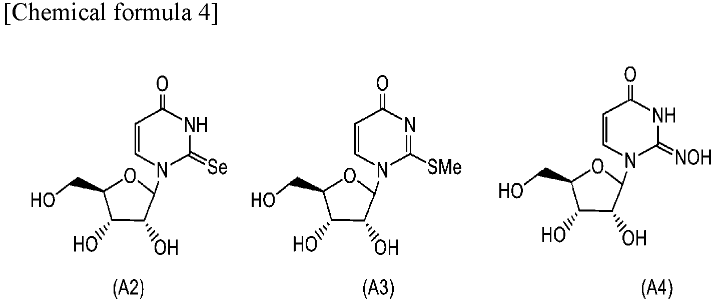
[5] The antiviral agent according to any one of [1] to [4], having an antiviral action against an enveloped virus having a positive-sense single-stranded RNA gene.
[6] A pharmaceutical composition containing the antiviral agent according to any one of [1] to [5] as an active ingredient.
[7] The pharmaceutical composition according to [6], used in the treatment or prevention of an infectious disease caused by an enveloped virus having a positive-sense single-stranded RNA gene.
[8] The pharmaceutical composition according to [6], used in the treatment of an infection caused by a coronavirus, flavivirus, or togavirus.
[9] The pharmaceutical composition according to [6], used in the treatment or prevention of COVID-19.
[10] A compound represented by any of formulas (A4) to (A7) shown below.

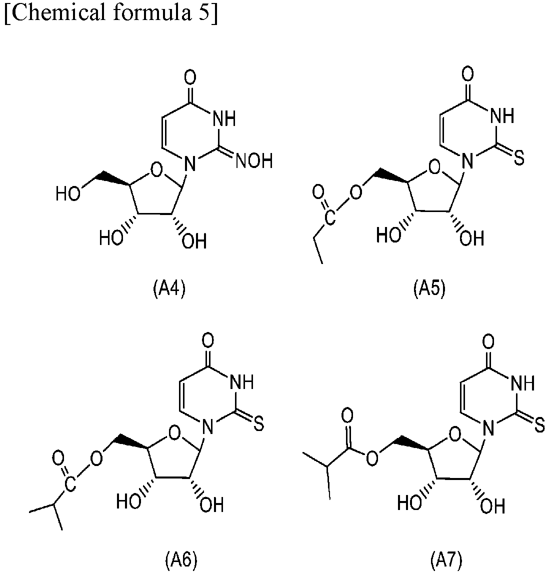

### [Advantageous Effects of Invention]

The antiviral agent according to the present invention has, as an active ingredient, a nucleic acid compound having high antiviral activity against enveloped viruses having a positive-sense single-stranded RNA gene such as coronaviruses and flaviviruses. Accordingly, the antiviral agent is ideal as an antiviral agent either in vivo or ex vivo, and a pharmaceutical composition containing the antiviral agent as an active ingredient is extremely useful as a pharmaceutical composition used in the treatment or prevention of infectious diseases caused by enveloped viruses having a positive-sense single-stranded RNA gene.

### [Brief Description of the Drawings]

FIG. 1A is a diagram illustrating the results of measuring the relative viral RNA level when cells infected with various strains of SARS-CoV-2 were subjected to 2-thiouridine treatment in Example 2.
FIG. 1B is a diagram illustrating the results of measuring the relative viral RNA level when cells infected with the SARS-CoV-2 omicron strain were subjected to 2-thiouridine treatment in Example 2.
FIG. 1C is a diagram illustrating the results of measuring the relative viral RNA level when cells infected with SARS-CoV-1 were subjected to 2-thiouridine treatment in Example 2.
FIG. 1D is a diagram illustrating the results of measuring the relative viral RNA level when cells infected with various strains of HCoV were subjected to 2-thiouridine treatment in Example 2.
FIG. 1E is a diagram illustrating the results of measuring the relative viral RNA level when cells infected with DENV2 were subjected to 2-thiouridine treatment in Example 2.
FIG. 1F is a diagram illustrating the results of measuring the relative viral RNA level when cells infected with ZIKV were subjected to 2-thiouridine treatment in Example 2.
FIG. 1G is a diagram illustrating the results of measuring the relative viral RNA level when cells infected with YFV were subjected to 2-thiouridine treatment in Example 2.
FIG. 1H is a diagram illustrating the results of measuring the relative viral RNA level when cells infected with JEV were subjected to 2-thiouridine treatment in Example 2.
FIG. 1I is a diagram illustrating the results of measuring the relative viral RNA level when cells infected with WNV were subjected to 2-thiouridine treatment in Example 2.
FIG. 1J is a diagram illustrating the results of measuring the relative viral RNA level when cells infected with CHIKV were subjected to 2-thiouridine treatment in Example 2.
FIG. 2A is a diagram showing the change over time in the survival rate (%) in groups of AG129 mice infected with AG129 mouse-adapted DENV2 and administered with 2-thiouridine in Example 3.
FIG. 2B is a diagram illustrating the results of measuring the serum viral RNA level 3 days post infection in groups of AG129 mice infected with AG129 mouse-adapted DENV2 and administered with 2-thiouridine in Example 3.
FIG. 3A is a diagram illustrating the results of measuring the virus titer (log₁₀(TCID₅₀)/mL) in the lungs of groups of mice infected with the MA-P10 strain of SARS-CoV-2 and administered with 2-thiouridine two hours prior to the virus infection in Example 5.
FIG. 3B is a diagram illustrating the results of measuring the viral RNA level (log₁₀(number of virus copies)/mL) in the lungs of groups of mice infected with the MAP10 strain of SARS-CoV-2 and administered intravenously with 2-thiouridine two hours prior to the virus infection in Example 5.
FIG. 4 is a diagram showing the change over time in the survival rate (%) in groups of mice infected with the MA-P10 strain of SARS-CoV-2 and administered intravenously with 2-thiouridine for a total of 5 days, including once two hours prior to the virus infection and then once per day from the day following infection until 4 days post infection, in Example 5.
FIG. 5 is a diagram illustrating the results of measuring the viral RNA level (log₁₀(number of virus copies)/mL) in the lungs of a group of mice infected with the MAP10 strain of SARS-CoV-2 and administered orally with 2-thiouridine following the virus infection in Example 5.

### [Description of Embodiments]

In the present invention and the present description, the expression "C_{y-z}" (wherein y and z are positive integers that satisfy y<z) means a number of carbon atoms of at least y but not more than z.

The antiviral agent according to the present invention is formed from a compound represented by general formula (A-1) or (A-2) shown below, a salt of the compound, or a solvate of the compound or salt.

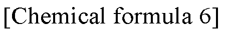

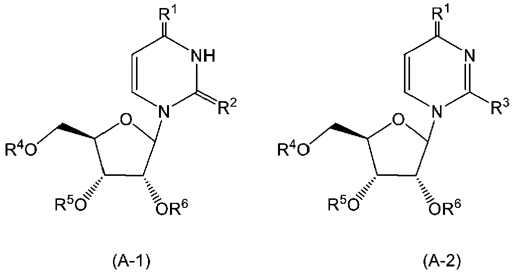

In general formula (A-1) or (A-2), R¹ represents an oxygen atom, sulfur atom, selenium atom, or =N-OR¹¹. R¹¹ represents a hydrogen atom, an alkyl group of 1 to 6 carbon atoms (a C₁₋₆ alkyl group ), or an aryl group.

When R¹¹ represents a C₁₋₆ alkyl group, the C₁₋₆ alkyl group may be either linear or branched. Examples of the C₁₋₆ alkyl group include a methyl group, ethyl group, propyl group, isopropyl group, butyl group, isobutyl group, sec-butyl group, tert-butyl group, pentyl group, isopentyl group, neopentyl group, tert-pentyl group, and hexyl group.

When R¹¹ represents a C₁₋₆ alkyl group, the C₁₋₆ alkyl group may have a substituent. A C₁₋₆ alkyl group having a substituent means a group in which one or a plurality of hydrogen atoms, and preferably 1 to 3 hydrogen atoms, bonded to a carbon atom of the C₁₋₆ alkyl group have each been substituted with another functional group. In the case the group has two or more substituents, the substituents may be the same or different. Examples of the substituent include an aryl group or a heteroaryl group.

When R¹¹ represents an aryl group, examples of the aryl group include a phenyl group, naphthyl group, anthryl group, and 9-fluorenyl group, but a phenyl group is particularly desirable.

When R¹¹ represents an aryl group, the aryl group may have a substituent. An aryl group having a substituent means a group in which one or a plurality of hydrogen atoms, and preferably 1 to 3 hydrogen atoms, bonded to a carbon atom of the aryl group have each been substituted with another functional group. In the case the group has two or more substituents, the substituents may be the same or different. Examples of the substituent include a C₁₋₆ alkyl group or a C₁₋₆ alkoxy group. The C₁₋₆ alkoxy group may be either linear or branched. Examples of the C₁₋₆ alkoxy group include a methoxy group, ethoxy group, propoxy group, butoxy group, tert-butoxy group, pentyloxy group, and hexyloxy group.

In general formula (A-1) or (A-2), R² represents an oxygen atom, sulfur atom, selenium atom, or =N-OR¹¹. R¹¹ is as described above. In general formula (A-1), R¹ and R² may be the same groups or different groups.

In general formula (A-2), R³ represents -O-R¹², -S-R¹³, or a guanidino group. R¹² and R¹³ each represent a C₁₋₆ alkyl group or an aryl group. When R¹² and R¹³ represents a C₁₋₆ alkyl group, the same groups as those described above for R¹¹ may be used as the C₁₋₆ alkyl group. When R¹² and R¹³ represents an aryl group, the same groups as those described above for R¹¹ may be used as the aryl group.

In general formula (A-1) or (A-2), R⁴ represents a hydrogen atom, -CO-R¹⁴, or - O-P(OH)(=O)-O-R¹⁵. R¹⁴ and R¹⁵ each independently represent an alkyl group of 1 to 30 carbon atoms (a C₁₋₃₀ alkyl group) or an aryl group.

When R¹⁴ and R¹⁵ represents an aryl group, the same groups as those described above for R¹¹ may be used as the aryl group.

When R¹⁴ and R¹⁵ represents a C₁₋₃₀ alkyl group, the alkyl group may be either linear or branched. Examples of the C₁₋₃₀ alkyl group include a methyl group, ethyl group, propyl group, isopropyl group, butyl group, isobutyl group, sec-butyl group, tert-butyl group, pentyl group, isopentyl group, neopentyl group, tert-pentyl group, hexyl group, isohexyl group, neohexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, heneicosyl group, docosyl group, tricosyl group, tetracosyl group, pentacosyl group, hexacosyl group, heptacosyl group, octacosyl group, nonacosyl group, and triacontyl group.

When R¹⁴ or R¹⁵ represents a C₁₋₃₀ alkyl group, one or more of the hydrogen atoms bonded to a carbon atom in the alkyl group may each be substituted with a phenyl group. R¹⁴ and R¹⁵ are each preferably a C₁₋₃₀ alkyl group with no phenyl group substitution, or a C₁₋₃₀ alkyl group in which one hydrogen atom has been substituted with a phenyl group.

When R¹⁴ and R¹⁵ has 2 or more carbon atoms, namely in the case of an alkyl group of 2 to 30 carbon atoms (a C₂₋₃₀ alkyl group), the group may have an oxygen atom of an ether bond between the carbon atoms. An "oxygen atom of an ether bond" is an oxygen atom that links two carbon atoms, and does not include groups in which oxygen atoms are linked directly in series.

In general formula (A-1) or (A-2), R⁵ and R⁶ each independently represent a hydrogen atom or -CO-R¹⁶. R¹⁶ represents a C₁₋₆ alkyl group or an aryl group. Examples of the C₁₋₆ alkyl group and the aryl group include the same groups as those listed above in relation to R¹¹.

The compound represented by general formula (A-1) or (A-2) is preferably a compound represented by any one of general formulas (1) to (9) shown below. In general formulas (1) to (9), R⁴ to R⁶ and R¹¹ are the same as R⁴ to R⁶ and R¹¹ in the general formula (A-1) or (A-2).

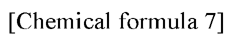

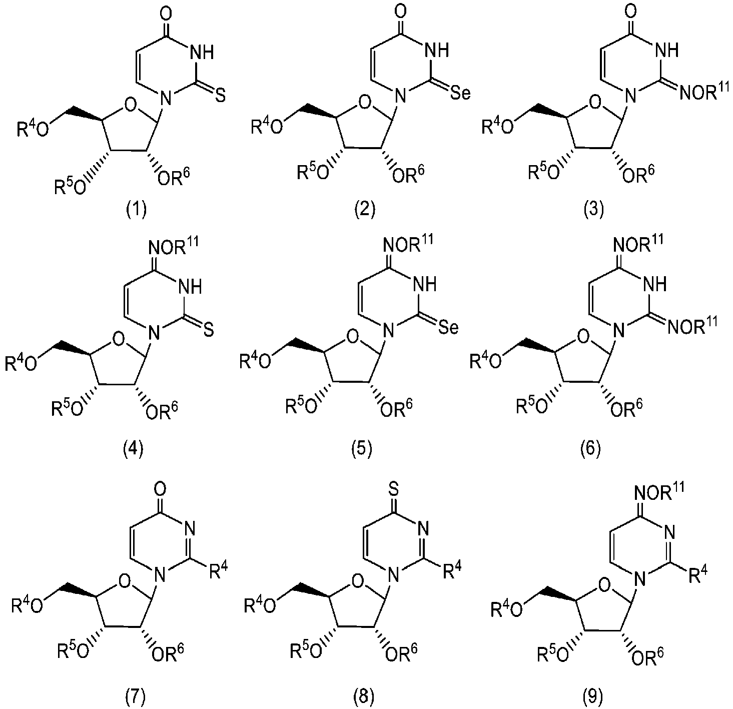

The active ingredient of the antiviral agent according to the present invention is preferably a derivative of the compound represented by general formula (A-1) or (A-2). The derivative of the compound represented by general formula (A-1) or (A-2) is preferably a derivative that produces the compound represented by general formula (A-1) or (A-2) when exposed to an enzymatic treatment or the like in vivo.

In particular, 2-thiouridine (CAS number: 20235-78-3) or a derivative thereof is preferred as the compound represented by general formula (A-1). The derivative of 2-thiouridine is preferably a derivative that produces 2-thiouridine when exposed to an enzymatic treatment or the like in vivo. In the present invention, derivatives of 2-thiouridine that can be used as the antiviral agent are more preferably derivatives obtained by the type of prodrug conversion used for drugs containing a nucleic acid compound as the active ingredient. Specific examples include derivatives in which a hydroxyl group bonded to the furan ring of the 2-thiouridine either directly or via the methylene group has been substituted with an acyl group or a phosphate group (PO₄⁻), monophosphoramidite derivatives in which the above hydroxyl group has been substituted with a monophosphoramidite group, and monophosphorothioate derivatives in which the above hydroxyl group has been substituted with a monophosphorothioate group. For example, examples of derivatives in which a hydroxyl group in 2-thiouridine (compound (A1)) has been acylated include compounds (A5) to (A8).

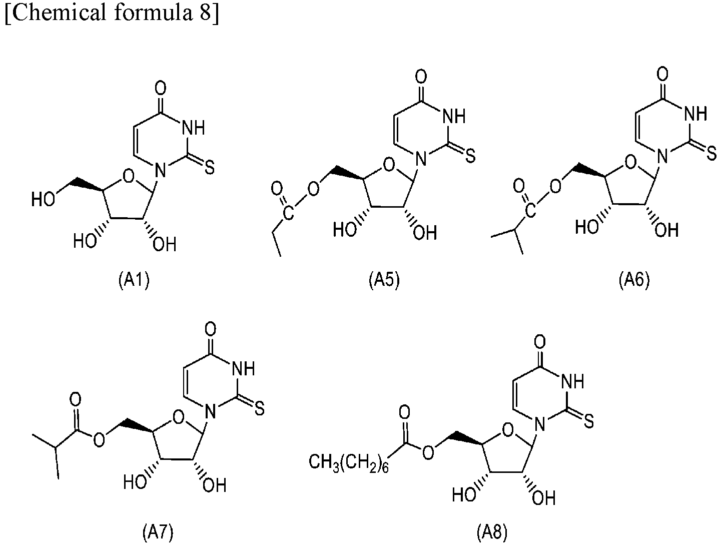

A compound represented by any of formulas (A2) to (A4) shown below or a derivative thereof is also preferred as the compound represented by general formula (A-1) or (A-2). These derivatives are preferably derivatives that produce the compound represented by any of formulas (A2) to (A4) when exposed to an enzymatic treatment or the like in vivo.

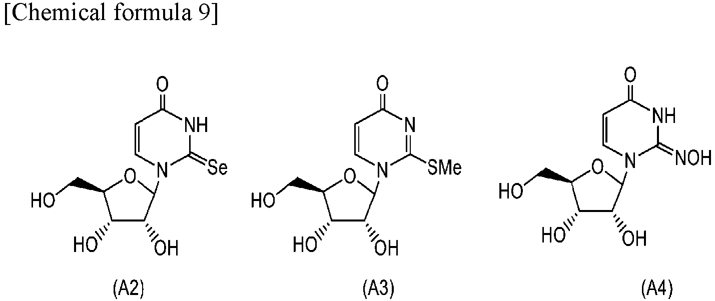

A compound represented by any of formulas (A9) to (A11) shown below or a derivative thereof is also preferred as the compound represented by general formula (A-1) or (A-2). These derivatives are preferably derivatives that produce the compound represented by any of formulas (A9) to (A11) when exposed to an enzymatic treatment or the like in vivo.

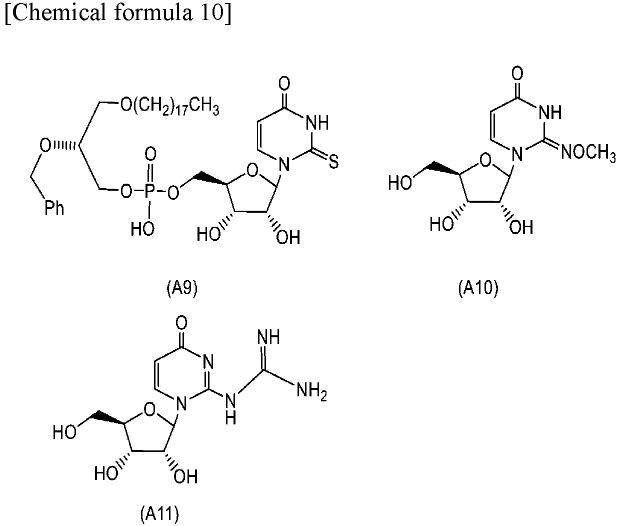

The compound represented by general formula (A-1) or (A-2) used as the active ingredient of the antiviral agent according to the present invention may be in the form of a salt, and examples of the acid or base used to form that salt include mineral acids such as hydrochloric acid and sulfuric acid; organic acids such as acetic acid, succinic acid, and citric acid; alkali metals such as sodium and potassium; and alkaline earth metals such as calcium and magnesium. Further, the compound represented by general formula (A-1) or (A-2) or the salt thereof may also exist in the form of a solvate such as a hydrate.

The compound represented by general formula (A-1) or (A-2), the salt of that compound, or the solvate of the compound or salt (hereinafter, these moieties are sometimes jointly referred to as the "nucleic acid compound according to the present invention") has an antiviral action against viruses, and particularly enveloped viruses having a positive-sense single-stranded RNA gene. Examples of enveloped viruses having a positive-sense single-stranded RNA gene include coronaviruses, flaviviruses, or togaviruses.

Examples of coronaviruses against which the nucleic acid compound according to the present invention exhibits antiviral activity include SARS-CoV-1, MERS-CoV, and SAR-CoV-2. Examples of flaviviruses against which the nucleic acid compound according to the present invention exhibits antiviral activity include the dengue virus (DENV) that causes dengue fever (dengue hemorrhagic fever), the Zika virus (ZIKV) that causes Zika fever (Zika virus infection), the yellow fever virus (YFV) that causes yellow fever, the West Nile virus (WNV) that causes West Nile fever (West Nile encephalitis), and the Japanese encephalitis virus (JEV) that causes Japanese encephalitis. There are four varieties of the DENV labeled from type 1 to type 4 (DENV1, DENV2, DENV3, DENV4). Examples of togaviruses against which the nucleic acid compound according to the present invention exhibits antiviral activity include the chikungunya virus (CHIKV) that causes chikungunya fever.

The nucleic acid compound according to the present invention is useful as the active ingredient of a pharmaceutical composition used in the treatment or prevention of virus infections, and particularly virus infections from enveloped viruses having a positive-sense single-stranded RNA gene. The pharmaceutical composition containing the nucleic acid compound according to the present invention as an active ingredient is preferably used for the treatment or prevention of infection by coronaviruses, flaviviruses or togaviruses, and in particular, is more preferably used for the treatment or prevention of SARS, MERS, COVID-19, dengue fever, Zika fever, yellow fever, West Nile fever, Japanese encephalitis, or chikungunya fever.

The nucleic acid compound according to the present invention is a low-molecular weight compound, and therefore does not suffer from problems of immunogenicity or the like. Further, the compound can be administered orally, and because there are few limitations on the administration pathway, the compound is very useful as the active ingredient for drugs designed for mammals including humans.

In those cases where one type, or two or more types, of the nucleic acid compound according to the present invention are included in a pharmaceutical composition, the compound(s) may be blended with a pharmaceutically acceptable carrier if necessary, and any of various dosage forms may be used depending on the purpose of the prevention or treatment. Examples of those dosage forms include oral agents, injections, suppositories, ointments, and patches. Oral agents are preferred. These dosage forms can be produced using production methods well-known to those in the field.

Examples of materials that may be used as pharmaceutically acceptable carriers include excipients, binders, disintegrants, lubricants and colorants used in solid formulations; and solvents, solubilizers, suspension agents, isotonicity agents, buffers, and pain relievers used in liquid formulations. Further, formulation additives such as preservatives, antioxidants, colorants, sweeteners, and stabilizers may also be used if necessary.

In the case of preparation of a solid formulation for oral administration, an excipient, and if necessary a binder, disintegrant, lubricant, colorant, and/or flavoring agent or the like are added to the nucleic acid compound according to the present invention, and a typical method may then be used to produce tablets, coated tablets, granules, powders, or capsules or the like.

In the case of preparation of a liquid formulation for oral administration, a sweetener, buffer, stabilizer, and/or flavoring agent or the like are added to the nucleic acid compound according to the present invention, and a typical method may then be used to produce an internal liquid formulation, syrup, or elixir or the like.

In the case of preparation of an injection, a pH regulator, buffer, stabilizer, isotonicity agent, and/or local anesthetic or the like are added to the nucleic acid compound according to the present invention, and a typical method may then be used to produce a subcutaneous, intramuscular, or intravenous injection.

In the case of preparation of a suppository, a formulation carrier known to those in the field, such as polyethylene glycol, lanolin, cacao butter, or a fatty acid triglyceride or the like, is added to the nucleic acid compound according to the present invention, and a typical method may then be used to produce the suppository.

In the case of preparation of an ointment, a typically used base agent, stabilizer, humectant and/or preservative or the like are added as necessary to the nucleic acid compound according to the present invention, and typical methods are then used to mix the ingredients and form the ointment.

In the case of preparation of a patch, an aforementioned ointment, or a cream, gel or paste or the like may be applied to a typical support using any typical method.

The amount of the nucleic acid compound according to the present invention within each of the formulations described above varies depending on the symptoms of the patient and the dosage form and the like, but typically, is within a range from about 0.001 to 1,000 mg in the case of an oral agent, within a range from about 0.001 to 500 mg in the case of an injection, and within a range from about 0.01 to 1,000 mg in the case of a suppository.

Furthermore, although the dosage per day for these formulations cannot be generalized, and varies depending on the symptoms, weight, age, and gender and the like of the patient, the dosage per day for a typical adult (weight: 60 kg) is within a range from about 0.005 to 5,000 mg, and preferably from 0.01 to 1,000 mg, and this dosage is preferably administered either once per day, or divided into about 2 or 3 doses per day.

There are no particular limitations on the animals that may be administered with the antiviral agent and the pharmaceutical composition containing the nucleic acid compound according to the present invention as an active ingredient, and the animal may be either a human or a non-human animal. Examples of these non-human animals include mammals such as cows, pigs, horses, sheep, goats, monkeys, dogs, cats, rabbits, mice, rats, hamsters and marmots, and birds such as chickens, quail and ducks.

### [Examples]

The present invention is described below in further detail using a series of examples, but the present invention is not limited to the following examples.

### <Coronaviruses>

Among the coronaviruses used in the tests described below, for the SARS-CoV-2 virus, the hCoV-19/Japan/TY/WK-521/2020 strain (SARS-CoV-2 Wuhan strain), the hCoV-19/Japan/QK002/2020 strain (SARS-CoV-2 alpha strain), the hCoV-19/Japan/TY8-612/2021 strain (SARS-CoV-2 beta strain), the hCoV-19/Japan/TY7-501/2021 strain (SARS-CoV-2 gamma strain), the hCoV-19/Japan/TY11-927/2021 strain (SARS-CoV-2 delta strain), and the hCoV-19/Japan/TY38-873/2021 strain (SARS-CoV-2 omicron (BA.1) strain) were used. For the SARS-CoV-1 virus, the Hanoi strain was used. The SARS-CoV-2 viruses used were distributed by the National Institute of Infectious Diseases, whereas the SARS-CoV-1 virus used was distributed by Nagasaki University of the National University Corporation.

For the coronaviruses that typically infect humans (Human Coronavirus: HCoV), the alpha coronavirus HCoV-229E strain obtained from ATCC (ATCC: VR-740) and the beta coronavirus HCoV-OC43 strain obtained from ATCC (ATCC: VR-1558) were used.

### <Flaviviruses>

Among the flaviviruses used in the tests described below, for DENV1 to DENV4, the D1/hu/PHL/10-07 strain, the D2/hu/INDIA/09-74 strain, the D3/hu/Thailand/00-40 strain, and the D4/hu/Solomon/08-11 strain respectively were used. For ZIKV, the MR766 strain was used. For YFV, the 17D-204 strain was used. For WNV, the NY99 strain was used. For JEV, the Beijing-1 strain was used. The YFV was distributed by Nagasaki University of the National University Corporation, whereas the remaining viruses were distributed by the National Institute of Infectious Diseases.

### <Togavirus>

For the togavirus used in the tests described below, the SL10571 strain was used for CHIKV This virus strain was distributed by the National Institute of Infectious Diseases.

### <Cultured Cells>

In the following tests, the cells used for infection with the various viruses included cells of the cultured cell line MRC5 derived from human fetal lungs, cells of the cultured cell line BHK-21 derived from Syrian hamster kidneys, cells of the cultured cell line VeroE6 derived from African green monkey kidneys, cells of the cultured cell line VeroE6/TMPRSS2 obtained by using gene recombination to express TMPRSS2 in VeroE6 cells, and 293T/ACE2 cells obtained by introducing the ACE2 gene into cells of the cultured cell line HEK293T derived from human embryonic kidneys and then expressing ACE2 homeostatically. For each of these cells, a 2% FBS/MEM medium was used as the culture medium. The 2% FBS/MEM was prepared by adding 2% of FBS (fetal bovine serum: manufactured by Gibco Inc.) and L-glutamine (manufactured by FUJIFILM Wako Pure Chemical Corporation) to MEM (Minimum Essential Medium: manufactured by Nissui Corporation).

### [Example 1]

Using the compounds of a compound library based on nucleic acid compounds held by the Center for Research and Education on Drug Discovery, Hokkaido University, National University Corporation as test samples, screening was conducted for compounds having an effect in inhibiting cell death of cells infected with a flavivirus. DENV2 was used as the flavivirus.

The results revealed that of the compounds included in the compound library, HUP0797 (2-thiouridine) exhibited antiviral activity.

### <Measurement of Antiviral Activity against Flavivirus>

Using 2-thiouridine, MTT assays were conducted using BHK-21 cells infected with eight different varieties of flavivirus (DENV1, DENV2, DENV3, DENV4, ZIKV, YFV, JEV, WNV) to ascertain the antiviral activity, namely, the inhibitory effect on cell death caused by virus infection.

### (1) Virus Infection and MTT Assay

First, in each well of a 96-well plate, cells that had been treated with one of the various compounds were infected with one of the various viruses, and culturing was then conducted in a CO₂ incubator (37°C, 5% CO₂), for 4 days in the case of DENV2, for 5 days in the case of DENV1, 3 and 4, and for 3 days in the case of ZIKV, YFV, WNV and JEV

The cultured 96-well plate was then inspected with the naked eye and under a microscope, and the state of the cells, and the presence or absence of crystals and the like were determined. Next, 30 µL of an MTT solution (a solution prepared by dissolving 3-(4,5-dimethyl-2-thiazol)-2,5-diphenyl-2H-tetrazolium bromide (manufactured by Nacalai Tesque Inc.) in PBS in sufficient amount to achieve a concentration of 5 µg/mL) was added to each well, and culturing was conducted for two hours in a CO₂ incubator. Subsequently, 150 µL of supernatant was removed from each well, with care taken not to remove the cells, and 150 µL of a cell lysate (a virus inactivation solution prepared by adding 50 mL of Triton X-100 and 4 mL of hydrochloric acid (12 N) to 500 mL of isopropanol) was then added to each well and mixed using a plate mixer. The absorbance of the wells of the 96-well plate was then measured at two wavelengths (570 nm and 630 nm) using an absorbance meter.

### (2) Calculation of EC₅₀

Based on the calculation formulas shown below, Microsoft Excel or another similar program having computational processing capabilities was used to calculate the 50% cell death inhibitory concentration (EC₅₀) of the virus infected cells.$\left[{EC}_{50}\right] = {10}^{Z}$ Z = ([50% OD] - [Low OD]) / ([High Od] - [Low OD]) × {log (High conc.) - log (Low conc.)} + log (Low conc.) $\left[50% OD\right] = \left\{OD \left(cell control\right)-OD \left(virus control\right)\right\} \times 0.5 + OD \left(virus control\right)$

OD: absorbance
conc.: drug concentration
OD (cell control): mean value of absorbance of control wells
OD (virus control): mean value of absorbance of virus control wells

EC₅₀ was calculated from the two points sandwiching the 50% OD value on the absorbance-drug concentration curve, namely A-High (High OD, High conc.) and B-Low (Low OD, Low conc.).

The results are shown in Table 1. As illustrated in Table 1, it was evident that 2-thiouridine exhibited high antiviral activity against DENV1, DENV2, DENV3, DENV4, ZIKV, YFV, WNV and JEV

**[Table 1]**

| | EC₅₀ [µM] | | | | | | | | CC₅₀ [µM] |
|---|---|---|---|---|---|---|---|---|---|
| Virus | DENV1 | DENV2 | DENV3 | DENV4 | ZIKV | YFV | JEV | WNV | BHK-21 |
| 2-thiouridine | 2.4 | 1.5 | 2.0 | 1.7 | 5.0 | 2.9 | 4.8 | 5.3 | >50 |

### <Measurement of Antiviral Activity against Coronaviruses and Togavirus>

MRC5 cells were infected with the HCoV-229E strain or the HCoV-OC43 strain, and BHK-21 cells were infected with CHIKV, and in each case the antiviral activity was investigated in the same manner as described above. The results are shown in Table 2.

**[Table 2]**

| | EC₅₀ [µM] | | | CC₅₀ [µM] |
|---|---|---|---|---|
| Virus | CHIKV | HCoV-229E | HCoV-OC43 | MRC5 |
| 2-thiouridine | 8.2 | 0.77 | 13 | >50 |

As illustrated in Table 2, it was confirmed that 2-thiouridine also exhibited antiviral activity against the togavirus CHIKV, and the coronaviruses of the HCoV-229E strain and HCoV-OC43 strain. In other words, 2-thiouridine has high antiviral activity against a broad range of enveloped viruses having a positive-sense single-stranded RNA gene.

### [Example 2]

The compound 2-thiouridine, for which antiviral activity has been confirmed in Example 1, was investigated for its inhibitory effect on the viral RNA replication of coronaviruses, flaviviruses and togaviruses.

The SARS-CoV-2 Wuhan strain, SARS-CoV-2 alpha strain, SARS-CoV-2 beta strain, SARS-CoV-2 gamma strain, SARS-CoV-2 delta strain, SARS-CoV-2 omicron strain and SARS-CoV-1 Hanoi strain were each used to infect VeroE6/TMPRSS2 cells. The HCoV-229E strain was used to infect MRC5 cells. The HCoV-OC43 strain, DENV2, ZIKV, YFV, JEV, WNV and CHIKV were each used to infect VeroE6 cells.

### <Confirmation of Viral RNA Replication Inhibitory Effect against Coronaviruses>

### (1) Viral Infection of Cultured Cells

Dilution of the test sample (2-thiouridine), each of the coronaviruses (SARS-CoV-2 Wuhan strain, SARS-CoV-2 alpha strain, SARS-CoV-2 beta strain, SARS-CoV-2 gamma strain, SARS-CoV-2 delta strain, SARS-CoV-2 omicron strain, SARS-CoV-1 Hanoi strain, HCoV-229E strain and HCoV-OC43 strain) and each of the cell varieties (VeroE6/TMPRSS2, MRC5, VeroE6) was conducted using a culture medium formed from 2% FBS/MEM.

First, either 100 µL or 300 µL of a cell solution that had been prepared with an appropriate cell count was injected into each well of a 96-well plate or 48-well plate, and the plate was cultured overnight in a CO₂ incubator (37°C, 5% CO₂). Next, the test sample that had been diluted in advance to a suitable concentration with the culture medium was added to each plate well containing the added cells (either 50 or 150 µL/well) so as to generate a doubling dilution series, and mixing was conducted using a plate mixer. Subsequently, 50 or 150 µL/well of a virus solution that had been diluted in advance to a suitable concentration with the culture medium was injected into each well of the plate containing the added test sample. The plate was then mixed using a plate mixer, and cultured for 24 to 48 hours in a CO₂ incubator (37°C, 5% CO₂).

### (2) RNA Extraction and Purification

Following culturing, the culture supernatant was removed from each well of the cultured plate, a Lysis buffer included with an RNA extraction kit (product name: PureLink Pro 96 total RNA Purification Kit, or PureLink RNA Mini Kit, manufactured by Thermo Fisher Scientific Inc.) was added to each well, and the resulting cell solution was collected. Subsequently, the total RNA was purified in accordance with the protocol included with the kit.

### (3) Measurement of Viral RNA

Using a Probe/Primer set designed for the nucleocapsid domain of each purified viral RNA, a qRT-PCR (real-time quantitative reverse transcription PCR) was conducted. The qRT-PCR was conducted using a commercially available kit (product name: EXPRESS One-Step SuperScript qRT-PCR kit, manufactured by Thermo Fisher Scientific Inc.) and a real-time PCR system (product name: QuantStudio 7 Flex Real-Time PCR System, manufactured by Thermo Fisher Scientific Inc.).

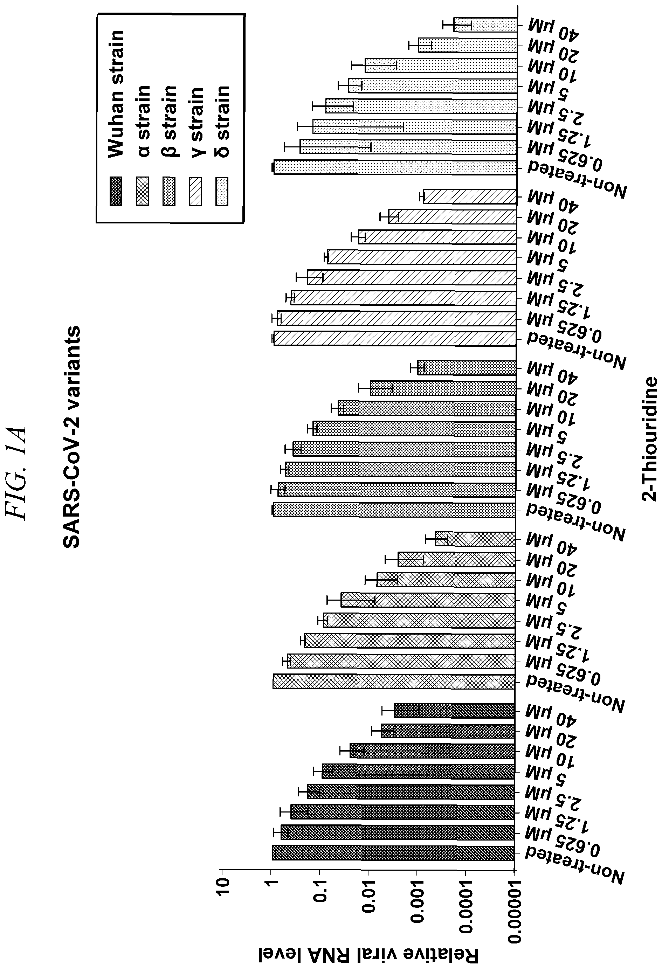

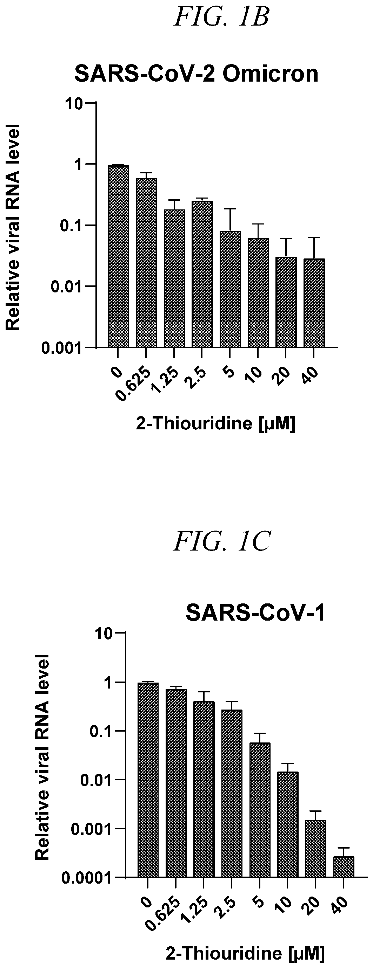

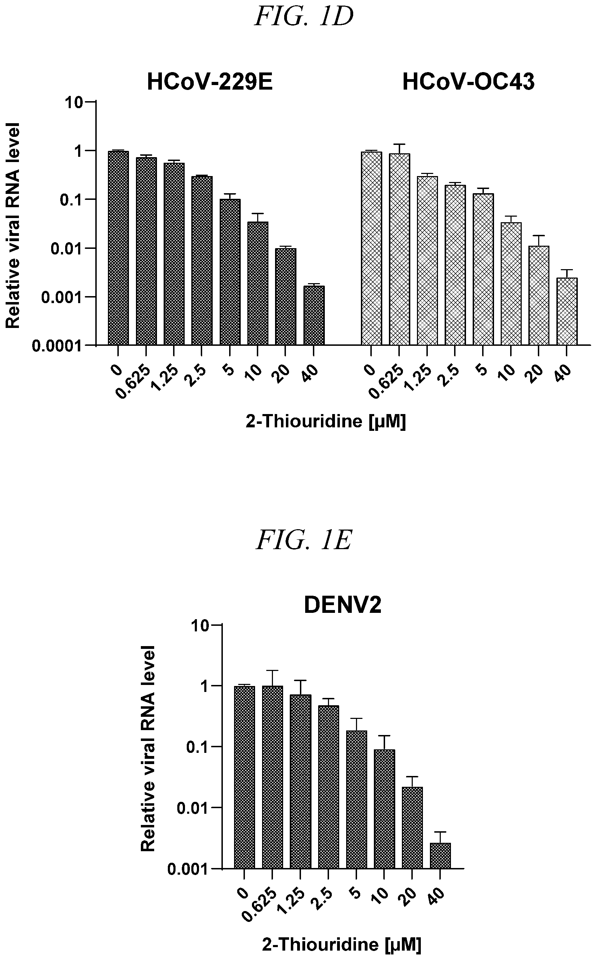

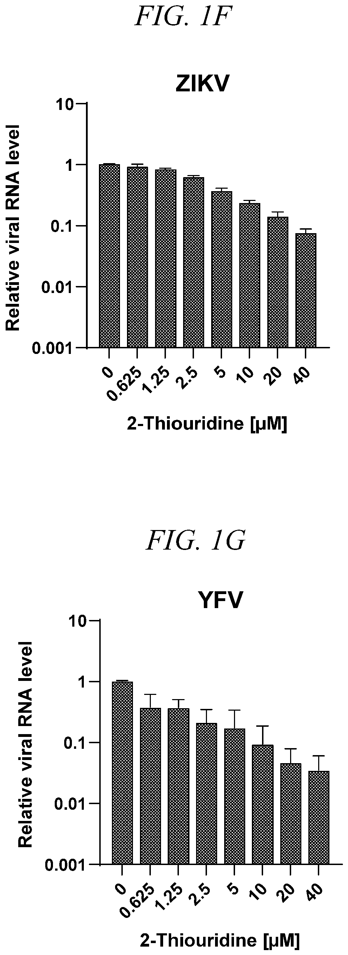

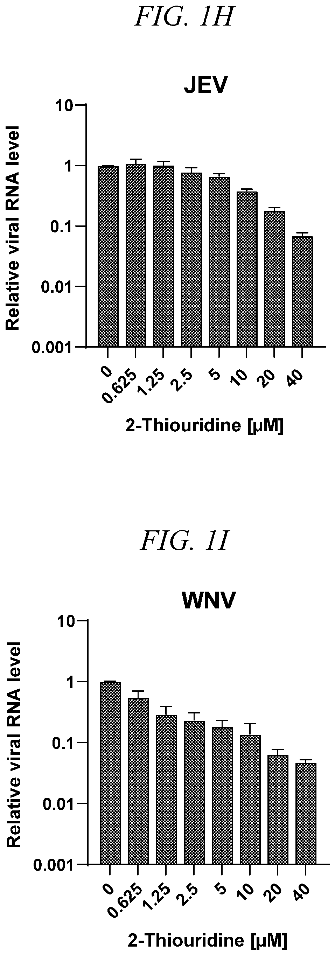

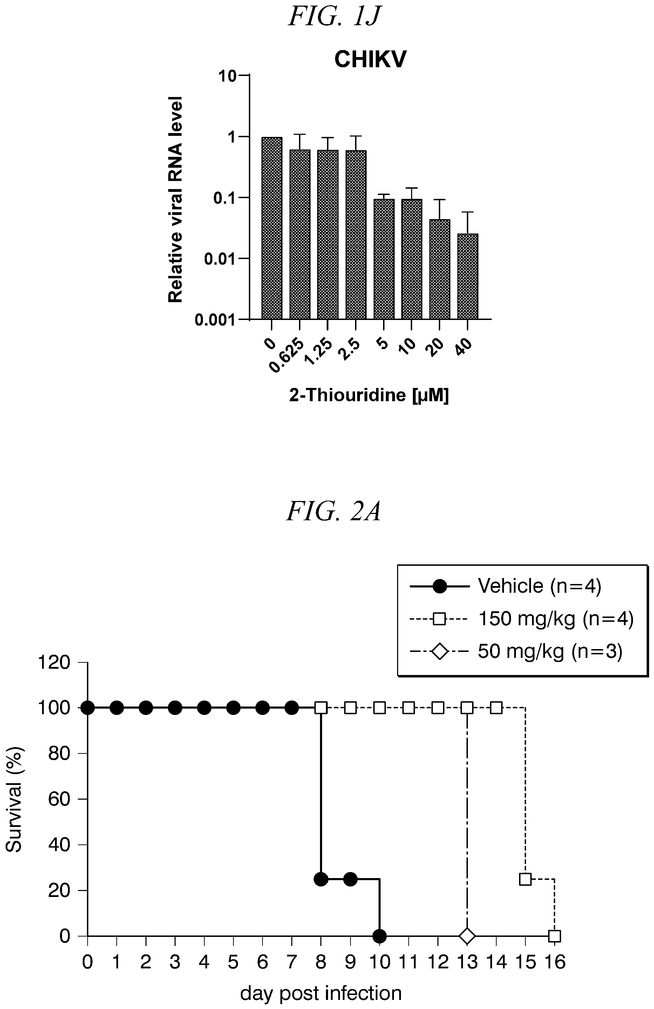

Using the ΔΔCt method, the viral RNA level in those cells to which 2-thiouridine had not been added was set to 1.0, and the relative viral RNA levels in the cells treated with the various concentrations of 2-thiouridine were calculated. At this time, the ACTB gene was used as an internal control. The results of measuring the relative viral RNA level in each of the infected cell samples are illustrated in FIG. 1A to FIG. 1D.

### <Confirmation of Viral RNA Replication Inhibitory Effect against Other Viruses>

With the exceptions of using DENV2, ZIKV, YFV, JEV, WNV and CHIKV as the viruses used for infection, using VeroE6 cells as the cells to be infected, and then conducting viral RNA extraction from the cells following culturing for 48 hours from virus infection in the case of DENV2, ZIKV, YFV and JEV, or for 24 hours from virus infection in the case of WNV and CHIKV, the viral RNA replication inhibitory effect of 2-thiouridine against each of these viruses was investigated in the same manner as described above. The results of measuring the relative viral RNA level in each of the infected cell samples are illustrated in FIG. 1E to FIG. 1J.

As illustrated in FIG. 1A to FIG. 1J, in the cells treated with 2-thiouridine, the viral RNA levels decreased in the cells infected with all of the different viruses. These results confirmed that 2-thiouridine has an inhibitory effect on the viral RNA replication of coronaviruses, flaviviruses and togaviruses, and exhibits good antiviral activity. These results indicate that 2-thiouridine exhibits broad antiviral activity against enveloped viruses having a positive-sense single-stranded RNA gene.

### [Example 3]

Mice infected with a flavivirus were treated with 2-thiouridine to investigate whether a therapeutic effect could be obtained. The test used AG129 mice lacking an interferon α-receptor and interferon γ-receptor, and a virus prepared by subculturing and adapting DENV2 for AG129 mice (namely, AG129 mouse-adapted DENV2). The 2-thiouridine was administered orally to the mice in the form of a solution diluted with an aqueous solution containing 5% DMSO and 0.5% MC (namely, an aqueous solution prepared by dissolving a solution of 2-thiouridine in 100% dimethyl sulfoxide (DMSO) (final concentration: 5%) in 0.5% methyl cellulose).

On the morning of the test start day, AG129 mice (7-week old female mice) were infected with the AG129 mouse-adapted DENV2 solution at a rate of 0.1 mL per mouse (1×10² PFU/mouse) by intraperitoneal administration. For the 5 day period from the day of virus infection (infection day 0) through until the fourth day post infection, the mice were administered orally twice per day with 2-thiouridine, once in the morning and once at night, using a per-weight dosage of either 50 mg/kg (50 mg/kg administration group, n=3) or 150 mg/kg (150 mg/kg administration group, n=4). On infection day 0, oral administration of the 2-thiouridine was conducted immediately following infection. As a control group, mice were administered orally in a similar manner with an aqueous solution containing 5% DMSO and 0.5% MC (Vehicle administration group, n=4).

The mice were observed every day following infection, and individuals in which the bodyweight dropped below 80% of the initial weight were deemed to have died. The results for the survival rate (%) of each group are shown in FIG. 2A. In the vehicle administration group, all of the mice had died within 10 days of the virus infection, whereas in the 50 mg/kg administration group, all of the mice survived until day 12 post various infection, and in the 150 mg/kg administration group, all of the mice survived until day 15 post virus infection.

Moreover, 5 additional mice were added to each of the 50 mg/kg administration group, the 150 mg/kg administration group and the vehicle administration group, and these mice were inoculated with AG129 mouse-adapted DENV2 and subjected to an infection test in the same manner as described above. The mice were observed every day following infection, and individuals in which the bodyweight dropped below 70% of the initial weight were deemed to have died. The results for the mice individuals detailed in FIG. 2A were also re-analyzed with the criteria for death adjusted to individuals in which the bodyweight dropped below 70% of the initial weight. The results for the survival rate (%) of each group obtained by adding the newly acquired data to the results of FIG. 2A are shown in Table. 3. In the vehicle administration group (n=9), all of the mice had died within 10 days of the virus infection, whereas in the 50 mg/kg administration group (n=8) and the 150 mg/kg administration group (n=9), the survival rate for the virus-infected mice increased in a dosage-dependent manner.

**[Table 3]**

| | Survival rate (%) | | |
|---|---|---|---|
| Days post infection | Vehicle administration group (n=9) | 50 mg/kg administration group (n=8) | 150 mg/kg administration group (n=9) |
| 0 | 100 | 100 | 100 |
| 1 | 100 | 100 | 100 |
| 2 | 100 | 100 | 100 |
| 3 | 100 | 100 | 100 |
| 4 | 100 | 100 | 100 |
| 5 | 100 | 100 | 100 |
| 6 | 100 | 100 | 100 |
| 7 | 100 | 100 | 100 |
| 8 | 66.67 | 100 | 100 |
| 9 | 44.44 | 100 | 100 |
| 10 | 0 | 100 | 100 |
| 11 | | 75 | 88.89 |
| 12 | | 50 | 88.89 |
| 13 | | 37.5 | 66.67 |
| 14 | | 37.5 | 66.67 |
| 15 | | 0 | 66.67 |
| 16 | | | 66.67 |

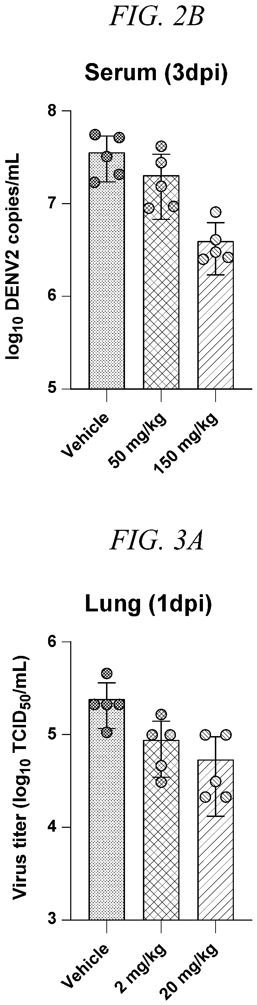

Furthermore, three days post infection, a blood sample was collected from each individual, and the viral RNA level in the serum (log₁₀(number of DENV2 copies)/mL) was measured (n=5 for each group). The measurement results are shown in FIG 2B. In the 50 mg/kg administration group and the 150 mg/kg administration group, the viral level in the blood decreased in a 2-thiouridine dosage-dependent manner compared from that observed for the vehicle administration group that was administered with only the medium and no drug (FIG. 2B).

These results indicated that 2-thiouridine has a death inhibitory effect on animals infected with dengue virus, and could be used as the active ingredient of a drug used for the treatment or prevention of infection by the dengue virus.

### [Example 4]

Structural analogs of 2-thiouridine were synthesized, and the antiviral activity of those analogs against the SARS-CoV-2 Wuhan strain was measured.

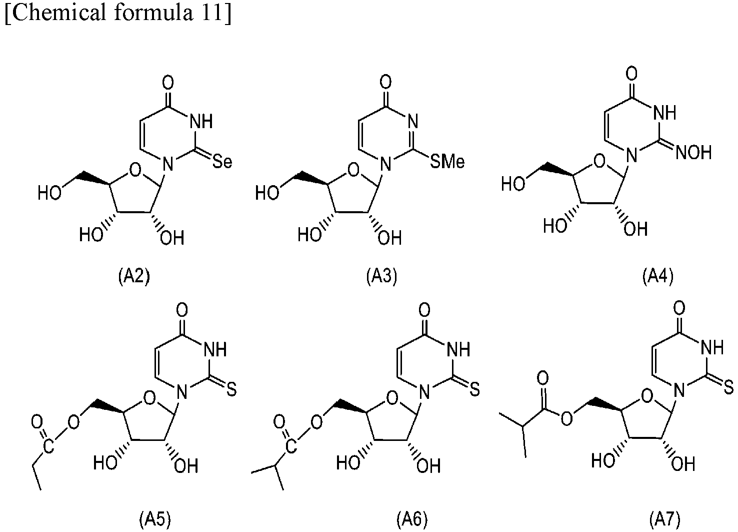

### (1) Compound (A2) and Compound (A3)

The compounds 2-selenouridine (compound (A2), CAS number: 40555-29-1) and 1-((3R,4S,SR)-3,4-dihydroxy-5-(hydroxymethyl)tetrahydrofuran-2-yl)-2-(methylthio)pyridin-4(1H)-one (compound (A3), CAS number: 175875-32-8) were synthesized using typical methods.

### (2) Compound (A4)

The compound 2-hydroxyisocytidine (compound (A4)) was synthesized in the following manner. First, 2,5'-anhydrouridine (CAS number: 22329-20-0) and hydroxylamine hydrochloride (153 mg, 2.21 mmol) were suspended in methanol (10 mL), triethylamine (308 µL) was added, and the mixture was microwaved at 70°C for one hour. Subsequently, the reaction liquid was returned to room temperature, the solvent was removed by distillation under reduced pressure, and the residue was crystallized from ethanol, yielding the compound (A4) (97 mg, yield: 84.8%).
ESI-MS m/z 260 (M+H)⁺
¹H-NMR (DMSO-d₆) (400 MHz) δ 9.53 (s, 1H), 9.36 (s, 1H), 7.84 (d, 1H, J=8.2 Hz), 5.54 (d, 1H, J=3.2 Hz), 5.28 (d, 1H, J=4.4 Hz), 5.12 (t, 1H, J=5.1, 5.4 Hz), 5.09-5.07 (m, 2H), 3.98-3.92 (m, 2H), 3.80 (ddd, 1H, J=3.2, 3.7, 5.6 Hz), 3.69 (ddd, 1H, J=3.2, 5.4, 12.4 Hz), 3.55 (ddd, 1H, J=2.7, 5.1, 12.4 Hz)

### (3) Compound (A5)

The compound 2',3'-O-isopropylidene-5'-O-propynyl-2-thiouridine (compound (A5)) was synthesized in the following manner. First, 2',3'-O-isopropylidene-2-thiouridine (isop-s2U) (CAS number: 6984-55-0) (200 mg, 0.67 mmol) was suspended in acetonitrile (CH₃CN) (5 mL), 4-dimethylaminopyridine (5 mg) and anhydrous propionic acid (130 µL, 1.5 equivalents) were added, and the resulting mixture was stirred at room temperature. After 30 minutes, methanol (1 mL) was added, and following stirring for a further 30 minutes, the solvent was removed by distillation under reduced pressure, and the residue was dissolved in ethyl acetate (10 mL) and then washed sequentially with water (5 mL), saturated sodium bicarbonate (5 mL) and water (5 mL). The thus obtained ethyl acetate layer was dried over anhydrous sodium sulfate, the solvent was removed by distillation under reduced pressure, and the resulting residue was dissolved in 98% formic acid (2 mL) and stirred for 6 hours at room temperature. Following this stirring, the reaction solution was concentrated under reduced pressure, and a process of adding water and conducting azeotropic distillation was repeated three times to remove the formic acid. The resulting residue was purified using a silica gel column (hexane : ethyl acetate = 1:1 to 1:3 (volumetric ratio)), yielding a foam-like compound (A5) (179 mg, yield: 85%).
ESI-MS m/z 316 (M+H)⁺
¹H-NMR (DMSO-d₆) (400 MHz) δ 7.75 (d, 1H, J=8.4 Hz), 6.56 (d, 1H, J=3.2 Hz), 6.03 (d, 1H, J=8.4 Hz), 5.55 (d, 1H, J=5.6 Hz), 5.32 (d, 1H, J=6.0 Hz), 4.24-4.32 (m, 2H), 4.05-4.09 (m, 2H), 3.90 (ddd, 1H, J=6.0 Hz), 2.38 (q, 2H, J=7.6 Hz), 1.04 (t, 3H, J=7.6 Hz)

### (4) Compound (A6)

The compound 2',3'-O-isopropylidene-5'-O-isobutyryl-2-thiouridine (compound (A6)) was synthesized in the following manner. First, isop-s2U (200 mg, 0.67 mmol) was suspended in acetonitrile (5 mL), 4-dimethylaminopyridine (5 mg) and anhydrous isobutyric acid (170 µL, 1.5 equivalents) were added, and the resulting mixture was stirred at room temperature. After 30 minutes, methanol (1 mL) was added, and following stirring for a further 30 minutes, the solvent was removed by distillation under reduced pressure, and the residue was dissolved in ethyl acetate (10 mL) and then washed sequentially with water (5 mL), saturated sodium bicarbonate (5 mL) and water (5 mL). The thus obtained ethyl acetate layer was dried over anhydrous sodium sulfate, the solvent was removed by distillation under reduced pressure, and the resulting residue was dissolved in 98% formic acid (2 mL) and stirred for 6 hours at room temperature. Following this stirring, the reaction solution was concentrated under reduced pressure, and a process of adding water and conducting azeotropic distillation was repeated three times to remove the formic acid. The resulting residue was purified using a silica gel column (hexane : ethyl acetate = 1:1 to 1:3 (volumetric ratio)), yielding a foam-like compound (A6) (197 mg, yield: 89.5%).
ESI-MS m/z 330 (M+H)⁺
¹H-NMR (DMSO-d₆) (400 MHz) δ 7.74 (d, 1H, J=8.2 Hz), 6.57 (d, 1H, J=3.2 Hz), 6.01 (d, 1H, J=8.2 Hz), 5.56 (d, 1H, J=5.6 Hz), 5.33 (d, 1H, J=6.0 Hz), 4.28 (d, 2H, J=3.6 Hz), 4.06-4.10 (m, 2H), 3.90 (ddd, 1H, J=6.0 Hz), 2.61 (sept, 1H, J=7.0 Hz), 1.11 (d, 6H, J=7.0 Hz)

### (5) Compound (A7)

The compound 2',3',5'-tri-O-isobutyryl-2-thiouridine (compound (A7)) was synthesized in the following manner. First, 2-thiouridine (130 mg, 0.5 mmol) was suspended in acetonitrile (10 mL), 4-dimethylaminopyridine (10 mg) and anhydrous isobutyric acid (375 µL, 2.4 mmol) were added, and the resulting mixture was stirred at room temperature. After 30 minutes, methanol (1 mL) was added, and following stirring for a further 30 minutes, the solvent was removed by distillation under reduced pressure, and the residue was dissolved in ethyl acetate (20 mL) and then washed sequentially with water (5 mL), saturated sodium bicarbonate (5 mL × 2) and water (5 mL). The thus obtained ethyl acetate layer was dried over anhydrous sodium sulfate, and the solvent was removed by distillation under reduced pressure, yielding a foam-like compound (A7) (230 mg, yield: 97.9%).
ESI-MS m/z 471 (M+H)⁺
¹H-NMR (CDCl₃) (400 MHz) δ 9.40 (br s, 1H), 7.72 (d, 1H, J=8.4 Hz), 6.86 (d, 1H, J=4.4 Hz), 6.02 (d, 1H, J=8.4 Hz), 5.41 (dd, 1H, J=4.4, 5.4 Hz), 5.26 (dd, 1H, J=5.5 Hz), 4.30-4.47 (m, 3H), 2.55-2.66 (m, 3H), 1.18-1.24 (m, 18H)

**[Table 4]**

| Compound | EC₅₀ [µM] | CC₅₀ [µM] | SI |
|---|---|---|---|
| A2 | 7.37 | >20 | >2.7 |
| A3 | 4.67 | 5.48 | 1.2 |
| A4 | 0.81 | 6.04 | 7.5 |
| A5 | 7.56 | 11.9 | 1.6 |
| A6 | 3.93 | 19.4 | 4.9 |

Measurement of the antiviral activity was conducted in the same manner as Example 1. The measurement results are shown in Table 4. In a similar manner to 2-thiouridine, all of the compounds exhibited antiviral activity against the SARS-CoV-2 Wuhan strain. Among these compounds, compounds (A2), (A4) and (A6) had satisfactory differences in the EC₅₀ and CC₅₀ concentration levels, indicating particular promise as antiviral agents.

### [Example 5]

Using a mouse model infected with a mouse-adapted strain of SARS-CoV-2 (derived from the MA-P 10 strain and WK-521 strain), the virus infection prevention effect and therapeutic effect of 2-thiouridine were investigated. The 2-thiouridine was injected intravenously into the mice as a solution diluted with physiological saline solution. Further, the 2-thiouridine was also administered orally to the mice in the form of a solution diluted with an aqueous solution containing 5% DMSO and 0.5% MC (namely, an aqueous solution prepared by dissolving a solution of 2-thiouridine in 100% DMSO (final concentration: 5%) in 0.5% methyl cellulose).

### (1) Virus Infection Prevention Effect of 2-thiouridine

A proliferation evaluation model using a SARS-CoV-2 MA-P10 strain-infected mouse model was used to investigate the virus proliferation inhibitory effect within the lungs of the mice generated by 2-thiouridine administration prior to virus infection.

First, 5-week old BALB/c mice were subjected to a single intravenous administration of 2-thiouridine (2 or 20 mg/kg) (n=5 for each of the 2 mg/kg administration group and the 20 mg/kg administration group). A control group was subjected to a similar single intravenous administration of physiological saline solution (vehicle administration group, n=5).

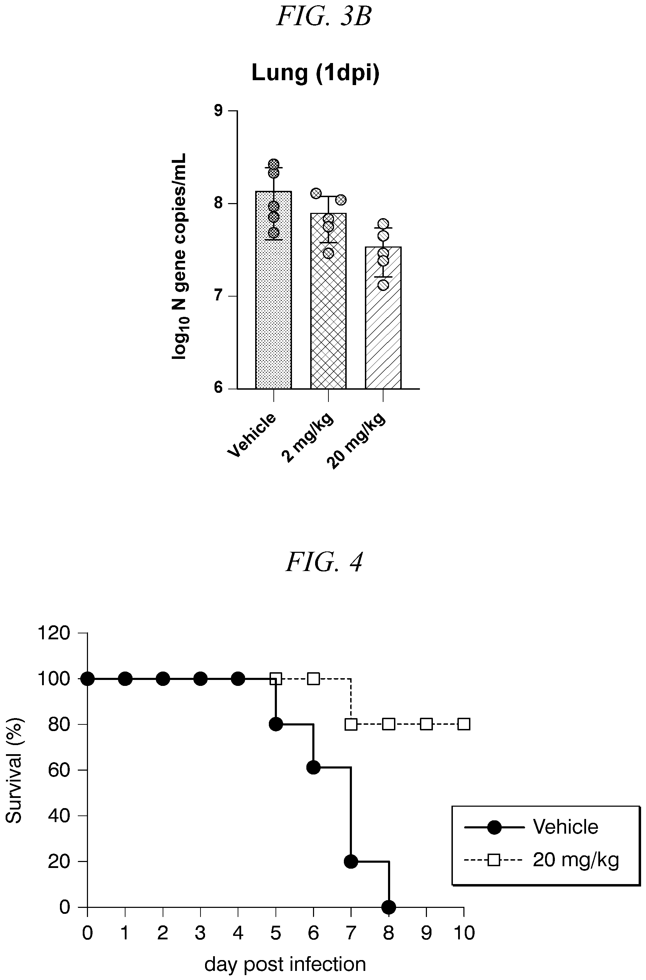

Two hours after the 2-thiouridine administration, each mouse was nasally inoculated with the MA-P10 strain (2×10² TCID₅₀/mouse), and on the day after infection, the lungs of each individual were collected, and the virus titer and viral RNA level in the lungs were measured. The measurement results (mean ± SD) for the virus titer (log₁₀(TCID₅₀)/mL) in the lungs of each group are shown in FIG. 3A, and the measurement results (mean ± SD) for the viral RNA level (log₁₀(number of virus copies)/mL) in the lungs are shown in FIG. 3B.

As illustrated in FIG. 3, in the mice that were subjected to a single intravenous administration of 2-thiouridine two hours before virus infection with SARS-CoV-2, the viral level in the lungs decreased in a 2-thiouridine dosage-dependent manner. These results confirmed that 2-thiouridine has a preventive effect on SARS-CoV-2 virus infection, namely, that 2-thiouridine holds promise as a prophylactic drug for COVID-19.

### (2) Death Inhibitory Effect of 2-thiouridine

Next, the death inhibitory effect of 2-thiouridine was investigated using the MAP10 strain-infected mouse model.

Specifically, ten 30- to 50-week old BALB/c mice were nasally inoculated with SARS-CoV-2 (MA-P10 strain) (2×10² TCID₅₀/mouse), and the survival rate (%) was evaluated for 10 days post virus infection. Half of the ten mice (namely, 5 mice) were administered intravenously (20 mg/kg) with 2-thiouridine for a total of 5 days, including once two hours prior to the virus infection and then once per day from the day following infection until 4 days post infection (20 mg/kg administration group). The remaining 5 mice were administered intravenously in a similar manner with physiological saline solution instead of 2-thiouridine (vehicle administration group).

The measurement results for the survival rate are shown in FIG. 4. In the 2-thiouridine administration group, the survival rate at day 10 post infection was 80%, whereas the survival rate for the mice in the vehicle administration group was 0% by day 8 post infection. In other words, it was confirmed that an inhibitory effect on death caused by SARS-CoV-2 could be obtained by repeated administration of 2-thiouridine.

### (3) Virus Infection Therapeutic Effect of 2-thiouridine

A proliferation evaluation model using the SARS-CoV-2 MA-P10 strain-infected mouse model was used to investigate the virus proliferation inhibitory effect within the lungs of the mice generated by 2-thiouridine administration post virus infection.

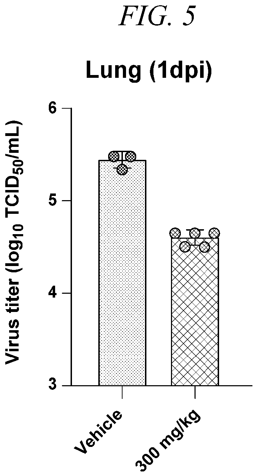

First, eight 5-week old BALB/c mice were nasally inoculated with the MA-P10 strain (2×10² TCID₅₀/mouse). Five of the eight mice were administered orally with 2-thiouridine from immediately after infection (twice per day, 300 mg/kg per administration) (300 mg/kg administration group). The remaining three mice were administered orally in a similar manner with an aqueous solution containing 5% DMSO and 0.5% MC instead of the 2-thiouridine (vehicle administration group). In this test, the 2-thiouridine was administered orally immediately after infection and then once 12 hours post infection, and 24 hours post infection, the lungs of each individual were collected, and the virus titer (log₁₀(TCID₅₀)/mL) in the lungs was measured. The measurement results (mean ± SD) for each group are shown in FIG. 5.

As illustrated in FIG. 5, in the mice administered orally with 2-thiouridine after virus infection with SARS-CoV-2, the virus titer in the lungs was markedly lower than that of the mice not administered with 2-thiouridine. These results clearly indicate that 2-thiouridine can reduce the viral level of SARS-CoV-2 in vivo even with administration post infection, and therefore that 2-thiouridine holds promise as a therapeutic drug for virus infection with SARS-CoV-2.

## Claims

1. An antiviral agent comprising a compound represented by general formula (A-1) or (A-2) shown below, a salt of the compound, or a solvate of the compound or salt: 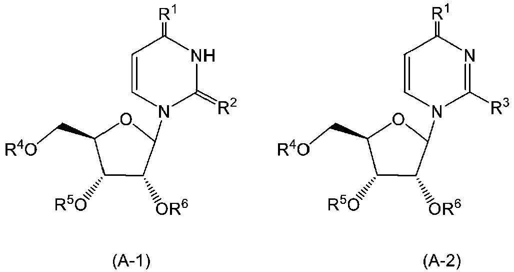 [wherein in the formulas, R¹ represents an oxygen atom, sulfur atom, selenium atom, or =N-OR¹¹; R² represents an oxygen atom, sulfur atom, selenium atom, or =N-OR¹¹; R³ represents -O-R¹², -S-R¹³, or a guanidino group; R¹¹ represents a hydrogen atom, an alkyl group of 1 to 6 carbon atoms, or an aryl group; R¹² and R¹³ each independently represent an alkyl group of 1 to 6 carbon atoms or an aryl group; R⁴ represents a hydrogen atom, - CO-R¹⁴, or -O-P(OH)(=O)-O-R¹⁵; R¹⁴ and R¹⁵ each independently represent an alkyl group of 1 to 30 carbon atoms which may be substituted with a phenyl group (and when the alkyl group has 2 or more carbon atoms, an oxygen atom of an ether bond may exist between the carbon atoms) or an aryl group; R⁵ and R⁶ each independently represent a hydrogen atom or -CO-R¹⁶; and R¹⁶ represents an alkyl group of 1 to 6 carbon atoms or an aryl group].

2. The antiviral agent according to Claim 1, wherein the compound represented by the general formula (A-1) or (A-2) is a compound represented by any of general formulas (1) to (9) shown below.

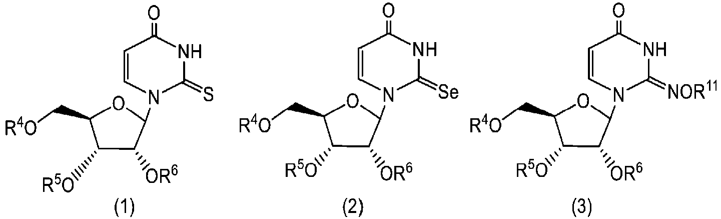

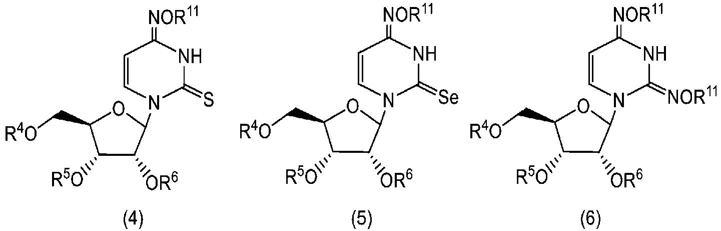

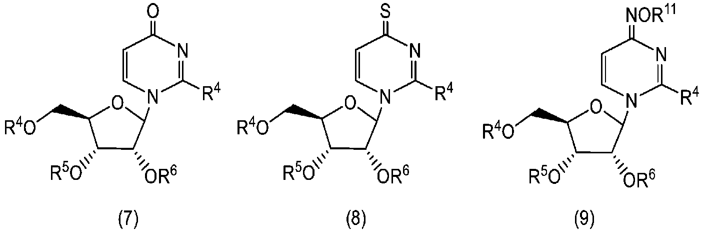

3. The antiviral agent according to Claim 1, wherein the compound represented by the general formula (A-1) or (A-2) is a compound represented by any of formulas (A1), (A5), (A6), (A7) and (A8) shown below.

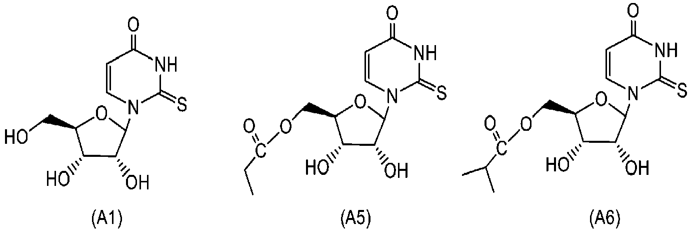

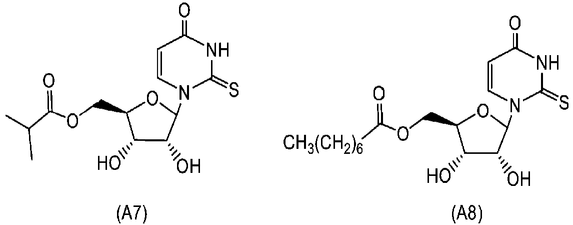

4. The antiviral agent according to Claim 1, wherein the compound represented by the general formula (A-1) or (A-2) is a compound represented by any of formulas (A2) to (A4) shown below.

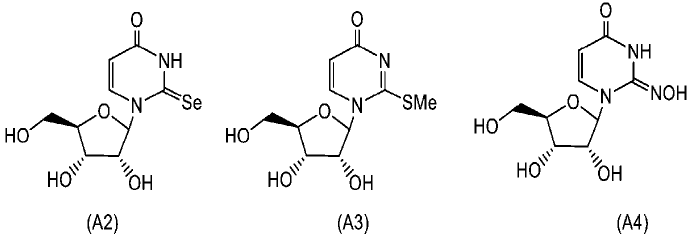

5. The antiviral agent according to any one of Claims 1 to 4, having an antiviral action against an enveloped virus having a positive-sense single-stranded RNA gene.

6. A pharmaceutical composition, comprising the antiviral agent according to any one of Claims 1 to 5 as an active ingredient.

7. The pharmaceutical composition according to Claim 6, used in treatment or prevention of an infectious disease caused by an enveloped virus having a positive-sense single-stranded RNA gene.

8. The pharmaceutical composition according to Claim 6, used in treatment or prevention of an infection caused by a coronavirus, flavivirus, or togavirus.

9. The pharmaceutical composition according to Claim 6, used in treatment or prevention of COVID-19.

10. A compound represented by any of formulas (A4) to (A7) shown below.
